# EUROPEAN PATENT APPLICATION

(11) **EP 4 497 443 A1**
(43) Date of publication of application: **29.01.2025**
(21) Application number: 23781114.6
(22) Date of filing: 31.03.2023
(51) Int. Cl.: A61K 45/00, A61K 31/7088, A61K 48/00, A61P 35/00, A61P 43/00, C12Q 1/02, C12Q 1/04, C12Q 1/68, G01N 33/15, G01N 33/48, G01N 33/50, C12N 15/113

(54) **THERAPEUTIC MEDICATION/PREVENTATIVE MEDICATION TARGETING SENESCENT CELLS OR SASP, METHOD FOR ACQUIRING DATA FOR DETECTING SENESCENT CELLS, AND SCREENING METHOD FOR THERAPEUTIC MEDICATION/PREVENTATIVE MEDICATION**

(30) Priority: 01.04.2022 JP 2022062157
(71) Applicant: Japanese Foundation For Cancer Research, Tokyo 135-8550 (JP)
(72) Inventor: MIYATA Kenichi, Tokyo 135-8550 (JP); TAKAHASHI Akiko, Tokyo 135-8550 (JP)
(74) Representative: Prüfer & Partner mbB Patentanwälte · Rechtsanwälte
(86) International application number: PCT/JP2023/013722
(87) International publication number: WO 2023/191106

(57) **Abstract**

It was revealed that by binding to CTCF which is important for maintaining chromosomal structures, hSATII RNA inhibits the function thereof, changes chromosome interaction, and induces transcription of inflammation-associated genes. It has also been discovered that cell death can be selectively induced in senescent cells and cancer cells by suppressing hSATII RNA expression. On the basis of the obtained results, a disease caused by cellular senescence can be treated with a substance that suppresses hSATII RNA or a substance that increases CTCF expression. Whether the disease is caused by cellular senescence can be diagnosed by measuring the expression of hSATII RNA and CTCF, and an epigenomic change of the hSATII DNA region. Thus, it is possible to provide a therapeutic drug for a disease caused by cellular senescence, a method for screening the therapeutic drug, and a method for acquiring data for detecting a disease caused by senescence.

## Description

### Technical Field

The present invention relates to a drug for use in treatment of a disease caused by senescent cells, via suppression of expression of an inflammation-associated gene targeting non-coding RNA expressed in senescent cells, or control of cell viability, a method for acquiring data for detecting senescent cells in a subject, and a method for screening a therapeutic drug/prophylactic drug.

### Background Art

When cells are placed under various stresses such as oxidative stress from active oxygen species and the like, damage to DNA by radiation, anticancer agents and the like, and activation of oncogenes, a state called "cellular senescence" occurs in which cell cycling irreversibly stops. Like the DNA repair mechanism and apoptosis, the cellular senescence functions as a suppression mechanism against a cancer that is caused by accumulation of genetic mutations caused by damage to DNA and mitochondrial insults. However, senescent cells accumulated in the body are known to highly express various inflammatory proteins, induce chronical inflammation with a senescence-associated secretory phenotype (SASP) secreted into peripheral tissues, and cause many diseases and pathological conditions associated with aging, such as cancer, neurodegenerative diseases, arterial sclerosis, Alzheimer's, lung fibrosis, osteoporosis and autoimmune diseases. Therefore, the control of senescent cells in the body or SASP may lead to suppression of development of diseases or treatment of diseases.

While the induction and control mechanisms for cellular senescence have not fully clarified yet, senolytic drugs selectively inducing cell death in senescent cells and senomorphic drugs suppressing SASP secreted by senescent cells may extend healthy life expectancy, and are widely expected to contribute to prevention of development and progress of pathological conditions associated with aging, and treatment of such conditions.

Patent Literature 1 discloses a complex for suppressing cellular senescence, by which a mitochondrial membrane potential reducing agent for removing dysfunctional mitochondria to suppress SASP and a p53 inhibitor are delivered together to cells of interest. Patent Literature 2 discloses a cellular senescence-suppressing agent capable of suppressing the senescence of stem cells.

However, the complex disclosed in Patent Literature 1 is intended to remove dysfunctional mitochondria associated with cellular senescence, and thus does not contribute to complete removal of senescent cells. The cellular senescence-suppressing agent disclosed in Patent Literature 2 is recognized to have an antioxidant action, but is not considered to be capable of eliminating cellular senescence.

### Citation List

### Patent Literature

Patent Literature 1: Japanese Patent Laid-Open No. 2021-24852
Patent Literature 2: Japanese Patent Laid-Open No. 2018-52879

### Non Patent Literature

Non Patent Literature 1: Casella G et al. Nucleic Acids Res 2019, Vol 47(14), pp. 7294-7305
Non Patent Literature 2: Ting T et al, Science, 2011, Vol.6017, pp.593-596. doi: 10.1126/science.1200801. Epub 2011 Jan 13.
Non Patent Literature 3: Kishikawa T et al, JCI Insight. 2016, Jun 2;1(8):e86646. doi: 10.1172/jci.insight.86646.
Non Patent Literature 4: Solovyov A Cell Rep, 2018, Vol.23(2), pp.512-521. doi: 10.1016/j.celrep.2018.03.042.
Non Patent Literature 5: Franses J W et al, Oncologist. 2018, Vol. 23(1): pp. 121-127, Published online 2017 Aug 31, doi:10.1634/theoncologist.2017-0234.
Non Patent Literature 6: Billingsley K J, Scientific Reports 2019, volume 9, Article number: 4369.
Non Patent Literature 7: Oezguer E et al. Clin Chim Acta. 2021, Vol.514, pp.74-79. doi: 10.1016/j.cca.2020.12.008. Epub 2020 Dec 31.
Non Patent Literature 8: Cambier L et al, Scientific Reports, 2021, vol. 11, Article number: 94.
Non Patent Literature 9: Nogalski, M T et al, Nature Communications. 2019, vol. 10, Article number: 90.
Non Patent Literature 10: Bersani F et al, PNAS, 2015, 112(49), pp.11548-15153. https://doi.org/10.1073/pnas.1518008112.
Non Patent Literature 11: Nogalski, M T et al, PNAS, 2020, Vol. 117 (50), pp. 31891-31901, https://doi.org/10.1073/pnas.2017734117.
Non Patent Literature 12: Kojima R et al., Nature Communications, 2018, vol.9, Article number: 1305(2018), DOI:10.1038/s41467-018-03733-8.

### Summary of Invention

### Technical Problem

As will be described in detail below, the present inventors have found that non-coding RNA which is not expressed in normal cells is highly expressed in senescent cells, and controls the expression of inflammatory genes. Further, it has been found that cell death can be induced in senescent cells by controlling the expression of non-coding RNA. Accordingly, an object is to provide a therapeutic drug which can target the non-coding RNA, suppress harmful SASP, and induce cell death in senescent cells to eliminate the senescent cells, and to provide Seno-therapy which is a new therapeutic method that targets cellular senescence. It has also been found that a cellular senescence state can be detected early in a subject by examining non-coding RNA that is a target, a molecule with which the expression changes, and epigenomes in the relevant gene region. Accordingly, another object is to provide a method for acquiring data which, when detected, indicates detected senescent cells, and hence contributes to diagnosis of a disease caused by senescent cells. A new target molecule for control of viability of senescent cells and SASP has been found. Accordingly, still another object is to provide a method for screening a therapeutic drug using the target molecule.

### Solution to Problem

The present invention relates to the following therapeutic drugs, methods for acquiring data, and methods for screening a therapeutic drug.
(1) A pharmaceutical composition which is a therapeutic drug and/or a prophylactic drug for a disease caused by senescent cells, the pharmaceutical composition suppressing expression or inhibiting activity of hSATII RNA, and/or increasing expression of CTCF.
   Increased expression of hSATII RNA and decreased expression of CTCF are closely related to cellular senescence. Therefore, a substance capable of regulating these enables treatment of a disease caused by cellular senescence.
(2) The pharmaceutical composition according to (1), wherein the pharmaceutical composition is a nucleic acid medicine which suppresses the expression or inhibits the activity of hSATII RNA and/or increases the expression of CTCF.
   In particular, as illustrated in the present specification, a nucleic acid or a compound that regulates the expression and the activity of hSATII RNA and CTCF is effective as a medicine.
(3) A method for acquiring data on whether cellular senescence occurs in a subject, comprising detecting any one of hSATII RNA, CTCF expression, and an epigenomic change of genomic DNA in a sample.
   A change in expression and activity of hSATII RNA and CTCF is a phenomenon specific to cellular senescence. Therefore, by identifying such a change in expression and activity, senescent cells can be detected. Before the expression of hSATII RNA increases and/or the expression of CTCF decreases in the process of inducing cellular senescence in normal cells or a cellular senescence-like phenotype in cancer cells, epigenomic changes such as swelling and increased chromatin accessibility of hSATII DNA region occur. Therefore, a method for detection thereof can be an effective test method for detecting senescent cells early.
(4) The method for acquiring data according to (3), wherein a disease caused by senescent cells is used in a test.
   If it is found that senescent cells are closely related to the disease, not only symptomatic therapy but also the pharmaceutical composition according to (1) or (2) can be expected to exhibit a high effect. An optimal therapeutic drug can be provided by examining whether the disease is caused by senescent cells.
(5) A method for screening a therapeutic drug and/or a prophylactic drug for a disease caused by cellular senescence, comprising adding a candidate compound to a medium for senescent cells, the candidate compound being selected at least one selected from the group of decreased expression of hSATII RNA, inhibition of functions of hSATII RNA, increased expression of CTCF, decreased expression of a SASP factor, shrinkage of the hSATII DNA region and decreased chromatin accessibility as an index.
   By using a change specific to cellular senescence as an index, a novel pharmaceutical composition can be screened. In particular, epigenomic changes such as increased expression of hSATII RNA and inflammatory SASP-associated genes, decreased expression of CTCF, and swelling and increased chromatin accessibility of the hSATII DNA region are phenomena specific to senescent cells. Therefore, detection of the changes enables screening of senolytic drugs and senomorphic drugs.
(6) A method for treating a disease caused by senescent cells, comprising acquiring data on whether the disease is caused by senescent cells, by the method according to (3) or (4), and administering the therapeutic drug according to (1) or (2) to treat the disease.
(7) A method for treating a disease caused by senescent cells, comprising administering a compound that suppresses expression or inhibits activity of hSATII RNA, and/or increases expression of CTCF.

### Brief Description of Drawings

[Figure 1A] (a) shows a scheme of screening of transcripts related to increased accessibility of chromatin, (b) shows a Volcano plot showing a change in ATAC-seq peak for IMR-90 cells in the proliferation phase and cells induced cellular senescence by an X-ray, and (c) shows 652 transcripts for which the accessibility of chromatin shown in (b) changes in the Volcano plot of RNA-seq data (GSE130727). In (b) and (c), the horizontal axis FC represents a fold change, and the vertical axis represents FDR.
[Figure 1B] Figure 1B shows the peaks in ATAC-seq and RNA-seq (GSE130727) data of the hSATII gene loci of IMR-90 cells in the proliferation phase and cells induced cellular senescence by an X-ray. The results of two biological replicates are shown. In each of ATAC-seq and RNA-seq, upper two rows and lower two rows represent the results obtained for IMR-90 cells in the proliferation phase and after cellular senescence induction, respectively.
[Figure 2A] Figure 2A is a heat map of SASP-associated gene expression in SVts8 cells and X-ray-induced senescent SVts8 cells in which centromere-derived non-coding RNA (hSATα) or pericentromere-derived non-coding RNA (hSATII) is overexpressed.
[Figure 2B] Figure 2B is a scattering diagram showing a change in accessibility of chromatin in SVts8 cells in which hSAT*α* (X axis) or hSATII (y axis) RNA is overexpressed, where vector-expressing cells are used for comparison.
[Figure 2C] Figure 2C shows enrichment analyses of gene sets related to senescence (upper) and inflammatory reaction (lower) in SVts8 cells in which hSATα or hSATII RNA is overexpressed.
[Figure 2D] Figure 2D is a diagram showing the results of RT-qPCR analysis of an effect of hSATII RNA knockdown on SASP gene expression in SVts8 cells of proliferation phase or SVts8 cells induced cellular senescence by an X-ray. *** P < 0.001.
[Figure 3A] Figure 3A is a diagram showing the results of analyzing an effect of hSATII RNA knockdown on SASP gene expression using skin fibroblasts derived from a healthy individual or a Werner syndrome patient.
[Figure 3B] Figure 3B is a diagram showing the results of analyzing an effect of hSATII RNA knockdown on SASP gene expression in oxidative stress-induced senescent cells using human umbilical vein endothelial cells.
[Figure 4A] Figure 4A shows the results of analysis of gene ontology of 280 hSATII RNA-binding proteins identified by mass analysis (left). Among them, 26 proteins are classified as chromatin-binding proteins (GO: 0003682). The top ten genes and the numbers of unique peptides are arranged on the right side.
[Figure 4B] Figure 4B is a diagram showing the results of performing a hSATα or hSATII RNA pull-down assay with a SVts8 cell lysate, and analyzing the binding with CTCF by Western blotting.
[Figure 4C] Figure 4C is a diagram showing the results of expressing CTCF with a FLAG tag (wild type), CTCF Δ*Z*F1-11 (mutant type) or CTCF ΔZF3-6 (mutant type) in HEK-293T cells, and analyzing the expression of each molecule by Western blotting.
[Figure 4D] Figure 4D is a diagram showing the results of RIP-qPCR analysis of binding of CTCF WT with a FLAG tag, CTCF ΔZF1-11 or ΔZF3-6, which is expressed in HEK-293T cells, to hSATII RNA. NS: Not significant, *** P < 0.001.
[Figure 5A] Figure 5A is a diagram showing the results of RT-qPCR analysis of an effect of hSATII RNA expression on the expression of a SASP-like inflammation-associated gene in SVts8 cells in which CTCF is forcibly expressed. The relative expression level is shown as a value obtained by normalization with respect to a value for vector-expressing cells. * P < 0.05, ** P < 0.01, *** P < 0.001.
[Figure 5B] Figure 5B is a diagram showing the results of RT-qPCR analysis of the expression of a SASP-like inflammation-associated gene in SVts8 cells in which expression of CTCF is suppressed by siRNA. The relative expression level is normalized with respect to a numerical value for cells treated with control siRNA. * P < 0.05, ** P < 0.01, *** P < 0.001.
[Figure 5C] Figure 5C is a diagram showing the results of RT-qPCR analysis of the expression of hSATII RNA, CTCF, cellular senescence and inflammation-associated genes of TIG-3 cells in which cellular senescence is induced by passage culture. The expression level of each gene is shown as a value obtained by normalization with respect to the expression level in the early stage of passage. *** P < 0.001.
[Figure 5D] Figure 5D is a diagram showing the results of Western blotting analysis of a decrease in amount of CTCF protein and expression of cellular senescence marker protein of TIG-3 cells in which cellular senescence is induced by passage culture.
[Figure 6A] Figure 6A is a Venn diagram showing a change in CTCF binding peaks which are based on ChIP-seq analysis.
[Figure 6B] Figure 6B shows a profile plot of signals in the region of center ± 2kb with regard to peaks obtained in ChIP-seq analysis (left), and a heat map divided into two clusters using the k-means algorism (right). The P value of the Wilcoxon rank-sum test is shown.
[Figure 6C] Figure 6C is a diagram showing the results of ChIP-qPCR analysis of an effect of hSATII RNA on CTCF binding to ICR located between IGF2 and H19.
[Figure 6D] Figure 6D is a diagram showing the results of EMSA analysis of an effect of hSATα or hSATII RNA on binding of CTCF to genomic DNA.
[Figure 6E] Figure 6E is a diagram showing RNA-seq, CTCF ChIP-seq and ATAC-seq profiles for gene loci of CXCL 10 and CXCL 11 that are typical SASP factors.
[Figure 6F] Figure 6F is a diagram showing the chromatin interaction reduced by hSATII RNA in the 3C-qPCR assay. The interaction between the constant region (C) and each target region (T) is shown. NS: Not significant, * P < 0.05.
[Figure 7A] Figure 7A is a diagram showing the results of RT-PCR experiment of the expression of hSATII RNA introduced into SVts8 cells by a retrovirus.
[Figure 7B] The left part of Figure 7B is a diagram showing the hSATII RNA expression increasing the number of multipolar cells. Microscopic images and the proportion of multipolar cells are shown. ** P < 0.01. The right part of Figure 7B is a diagram showing the hSATII RNA expression increasing the number of cells in which chromosome bridges are formed. A microscopic image and the proportion of multipolar cells are shown. *** P < 0.001.
[Figure 7C] Figure 7C is a diagram showing the results of karyotype analysis of SVts8 cells in which hSATII RNA is overexpressed (n = 20). *** P < 0.001.
[Figure 7D] Figure 7D is a diagram showing the scaffold-independent proliferation ability of SVts8 cells in which hSATII RNA is overexpressed. * P < 0.05.
[Figure 7E] The left part of Figure 7E shows immunostaining images showing an effect of hSATII RNA expression on multi-polarization in SVts8 cells in which CTCF is overexpressed. The right part of Figure 7E is a diagram showing proportions of multipolar cells arose by hSATII RNA expression in SVts8 cells in which CTCF is overexpressed. * P < 0.05, ** P < 0.01.
[Figure 8A] Figure 8A is a diagram showing the results of RT-PCR experiment of the expression of MajSAT RNA introduced into mouse embryonic fibroblasts MEFs by a retrovirus.
[Figure 8B] The left part of Figure 8B is a diagram showing the MajSAT RNA expression increasing the number of multipolar cells. Microscopic images and the proportion of multipolar cells are shown. * P < 0.05. The right part of Figure 8B is a diagram showing the MajSAT RNA expression increasing the number of cells in which chromosome bridges are formed. Microscopic images and the proportion of cells in which chromosome bridges are formed are shown. * P < 0.05.
[Figure 8C] Figure 8C is a diagram showing the scaffold-independent proliferation ability of MEFs in which MajSAT RNA is overexpressed. *** P < 0.001.
[Figure 8D] Figure 8D is a diagram showing the results of karyotype analysis of MEFs in which MajSAT RNA is overexpressed (n = 20). *** P < 0.001.
[Figure 8E] MEFs in which MajSAT RNA was overexpressed were implanted under the skin of nude mice. Figure 8E shows the weights (left) and pictures (right) of tumors on Day 20 (MEF/MajSAT RNA #2) or Day 30 (MEF/Vector, MEF/MajSAT RNA #1, MEF/MajSAT RNA #3). ** P <0.01.
[Figure 9A] Figure 9A shows the results of RT-qPCR analysis of hSATII and hSATα RNA in extracellular vesicles derived from RPE-1/hTERT cells. * P < 0.05, *** P < 0.001.
[Figure 9B] The expression of a SASP-like inflammation-associated gene by designer exosomes formed by an EXOtic device was evaluated by RT-qPCR. Each value was normalized with EXOtic-Nluc-treated cells.
[Figure 9C] Figure 9C shows box plots of the quantified results of RNA-ISH using a hSATII RNA probe in colorectal cancer specimens. The hSATII RNA positive cell ratios in normal epithelial cells and tumor cells (left), and normal fibroblasts and cancer-associated fibroblasts (right) are shown. The upper and lower hinges denote third and first quartiles, respectively, the upper and lower whiskers denote the maximum value and the minimum value, respectively, within a range of 1.5 times of the quartile, and the horizontal line in the box denotes a median value. ** P < 0.01, *** P < 0.001.
[Figure 9D] Figure 9D is a diagram schematically showing a mechanism in which the increased expression of pericentromere-derived hSATII RNA due to cellular senescence induces the expression of a SASP-associated inflammation gene.
[Figure 10A] In human pancreatic stromal cells in which cellular senescence was induced, hSATII expression was suppressed by siRNA. Figure 10A is a diagram showing the results of analyzing the effect.
[Figure 10B] Figure 10B is a diagram showing induction of cell death or suppression of proliferation by suppressing hSATII expression in senescent fibroblasts, immortalized cells, and cancer cells in which hSATII is highly expressed.
[Figure 11A] Figure 11A shows the compared results of analyzing the opened state of the hSATII DNA region by DNA-FISH and gene expression by RT-qPCR at each number of passages in induction of cellular senescence in TIG-3 cells through passage culture. NS: Not significant, *** P < 0.001.
[Figure 11B] Figure 11B is a scattering diagram obtained by plotting the chromatin accessibility of the hSATII DNA region from DNaseI sequence data of various kinds of cancer cell lines.
[Figure 12] Figure 12 is a diagram schematically showing an aspect of screening.
[Figure 13] Figure 13 is a diagram schematically showing the function of hSATII RNA, and the concept of Seno-therapy which targets hSATII RNA.

### Description of Embodiments

The present invention will be described in detail below showing data, but the present invention is not limited thereto.

The therapeutic drugs shown below are effective against age-related diseases and inflammatory diseases and pathological conditions. The age-related disease and pathological condition can include all diseases and pathological conditions developing due to aging. Examples thereof include cancers in which the number of patients increases with aging, such as breast cancer, colorectal cancer and pancreatic cancer, in particular, inflammatory cancer, cardiovascular diseases, Alzheimer's disease, neurodegenerative diseases such as Parkinson's, osteoporosis, gonarthrosis, ocular degenerative diseases such as glaucoma, cataract and maculopathy, chronic obstructive pulmonary disease, chronic renal disease, inflammatory diseases such as autoimmune diseases and psoriasis, sarcopenia, hepatic diseases such as fatty liver caused by obesity, and hepatic cirrhosis, diabetes, and enlarged prostate. The therapeutic drugs are effective against not only inflammatory diseases and pathological conditions developing due to aging, but also infectious diseases with inflammation, for example, inflammation caused by infectious diseases such as COVID-19, and for improvement of efficiency during implantation of stem cells by suppressing cellular senescence.

The therapeutic drugs shown below target non-coding RNA expressed in senescent cells, and thus can suppress not only an already developed disease, but also development of disease due to cellular senescence. Therefore, the therapeutic drugs are also effective in prevention of disease development, prevention of recurrence and the like. For the administration method, administration can be performed by an administration method commonly used for the diseases.

The term "cellular senescence" (sometimes referred to simply as "senescence") refers to a state in which a continuous DNA damage response irreversibly stops the proliferation of normal cells. A cell undergoing cellular senescence is referred to as a senescent cell, and secretes SASP factors such as inflammatory protein and extracellular vesicles. The term "cellular senescence-like" refers to a state in which in normal cells or senescent cells, the expression of inflammatory genes increases, and inflammatory proteins, extracellular vesicles and the like are secreted. The concepts of the cellular senescence and the aging of human or animal individuals are different. The aging of individuals corresponds to physical age, whereas the cellular senescence corresponds to biological aging, where the number of senescent cells increases depending on how much stress has been placed on the cells.

Substances secreted as SASP factors include inflammatory proteins such as inflammatory cytokines, chemokines, matrix metalloproteinases and growth factors, and extracellular vesicles such as exosomes. The inflammatory proteins specifically include inflammatory cytokines such as TNF-α, IL-1 and IL-6, CC chemokines, among which CCL-1 to CCL28 have been identified, CXC chemokines, among which CXCL1 to CXCL17 have been identified, C chemokines including XCL1 and XCL2, CX3C chemokines including CX3CL1, matrix metalloproteinases, among which MMP1 to MMP28 have been identified, and growth factors such as TGF-β, BMP, FGF, PDGF and HGF. In addition, there can be various substances that are secreted in association with inflammation.

Whether the disease or pathological condition is one on which the therapeutic drug of the present invention exhibits an effect can be determined by tests that will be described in detail below. Conducting a test beforehand to determine whether the present therapeutic drug exhibits an effect is important not only in selection of an appropriate therapeutic method for a patient but also in health economics.

### [Analysis of transcript in which expression changes in senescent cells]

Therapeutic targets for cellular senescence will be described below showing data. The experiment techniques used hereinafter, such as assay methods, follow methods commonly carried out in the art unless otherwise specified.

It has been reported that cells which have undergone cellular senescence show abnormal karyomorphisms, where expression of an inflammatory gene is induced via activation of cytosolic DNA sensing pathways mechanism. Further, it is known that a dramatical change is induced in a chromatin structure by cellular senescence, so that gene expression changes. Thus, for the purpose of clarifying the mechanisms of changes in chromatin structure and gene expression in the process of cellular senescence, changes in chromatin accessibility and RNA expression in senescent cells and normal cells were compared.

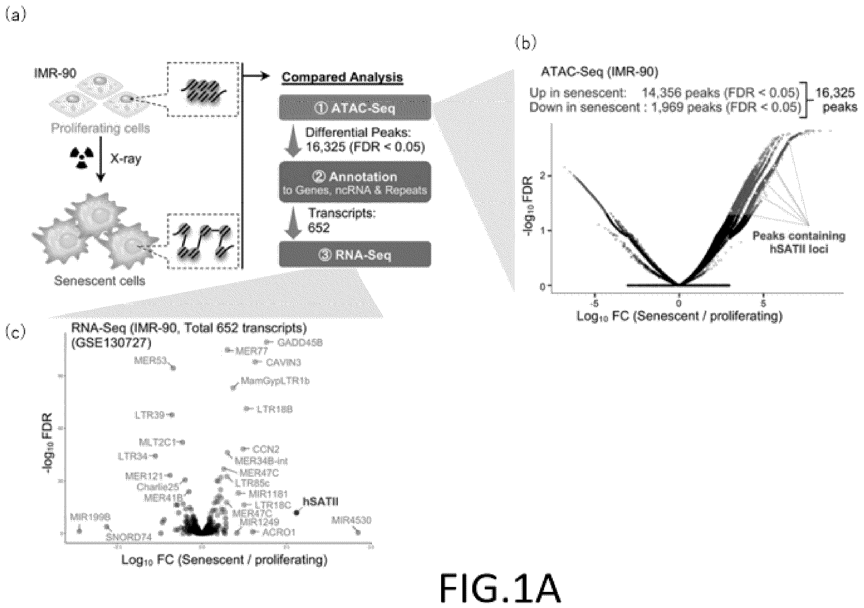

Using cells in proliferation phase and cells induced cellular senescence by an X-ray of human normal diploid fibroblasts IMR-90 (obtained from ATCC), ATAC-seq (assay for transposase-accessible chromatin sequencing) was performed. It was revealed that 16325 regions (false discovery rate < 0.05) changed in the process of cellular senescence (Figure 1A, (a)).

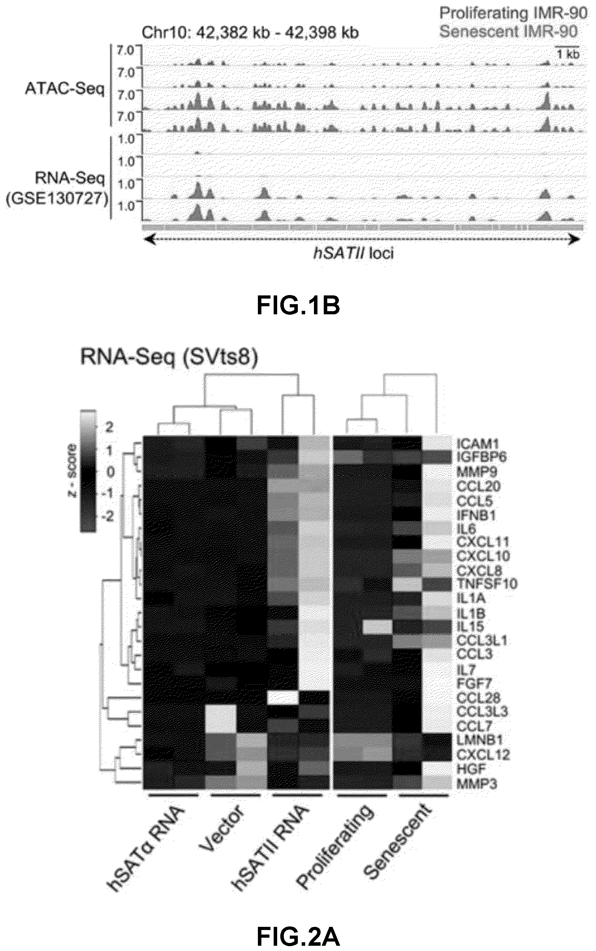

The results of ATAC-seq showed that for IMR-90 in which cellular senescence had been induced by an X-ray, there were 14356 peaks corresponding to an increase in chromatin accessibility and 1969 peaks corresponding to a decrease in chromatin accessibility (Figure 1A, (b)). For 16325 regions where chromatin accessibility changed, 652 transcripts were annotated using databases including GRCh37/hg19 and RepeatMasker.

Reanalysis was performed on the 652 transcripts using data in which the expression levels of the transcripts are published (GSE130727, Non Patent Literature 1) (Figure 1A, (c)). The expression levels were analyzed using RNA-seq data (GSE130727) of the IMR-90 cells in the proliferation phase and in senescent phase induced by an X-ray. The results revealed that for the IMR-90 cells in which cellular senescence had been induced by an X-ray, the accessibility of hSATII (human satellite II) gene loci which are repeated sequences present in pericentromeric regions was high (Figure 1A, (b)), and the expression thereof markedly increased as compared to that in the cells in the proliferation phase (logic fold change = 2.8, Figure 1A, (c)).

Combined ATAC-seq data and RNA-seq data (GSE130727) of IMR-90 cells in the proliferation phase and IMR-90 cells in which cellular senescence had been induced by an X-ray show that senescent cells had higher chromatin accessibility of hSATII gene loci, and an increased number of transcripts as compared to proliferative cells (Figure 1B). Although data are not shown, hSATII RNA expression associated with cellular senescence was detected even in IMR-90 cells in which cellular senescence had been induced by passage culture or the oncogene H-Ras^{V12} had been expressed, and normal diploid fibroblasts TIG-3 which had continuously undergone passage culture for a long period.

For hSATII RNA, enhancement of RNA transcription is observed in pancreatic cancer cells (Non Patent Literature 2), and hSATII RNA has been reported to be highly expressed in many cancers such as colorectal cancer, and Parkinson's disease and viral infection diseases, and shown to be a possible diagnostic marker for cancer (Non Patent Literatures 3 to 9). Further, it is indicated that proliferation of cancer cells and tumors is suppressed using LNA targeting hSATII RNA (Non Patent Literature 10 and 11). However, the fact that hSATII is expressed in senescent cells and controls the expression of inflammatory genes was found for the first time by the present inventors.

### [Change given by hSATII RNA expression]

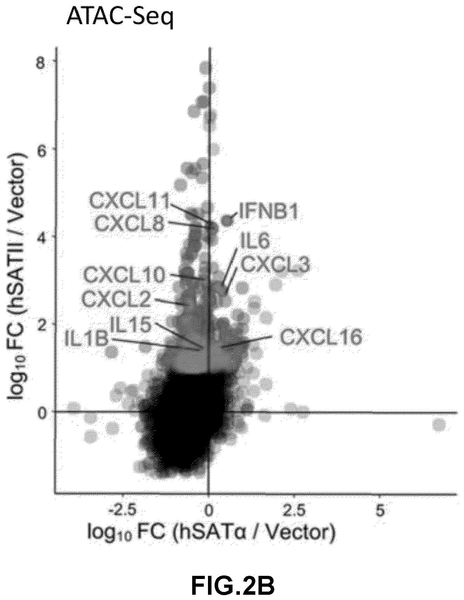

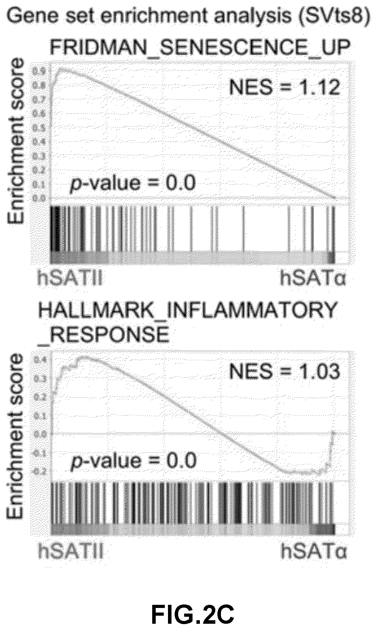

Analysis was performed on what kind of effect hSATII RNA expression had in a biological sense. With SVts8 cells which are an immortalized human fibroblast line (a cell line obtained by transfecting TIG-3 cells with a temperature-sensitive SV40-Large T antigen gene), hSATII RNA was overexpressed. The results of RNA-Seq showed that the overexpression of hSATII RNA induced the expression of SASP-like inflammatory genes. On the other hand, the overexpression of human satellite α RNA (hSATα) located in the centromeric region did not lead to the expression of inflammatory genes (Figure 2A). Also, the regions of inflammatory genes were shown to be opened by the overexpression of hSATII RNA, but were not opened in the case of hSATα RNA (Figure 2B). The analysis of the expression of genes related to inflammatory reaction and SASP suggested that the overexpression of hSATII RNA increased the expression of SASP-like inflammatory genes (Figure 2C).

Further, in SVts8 cells of the proliferation phase, and in SVts8 cell of cellular senescence had been induced by an X-ray, hSATII RNA was knocked down with siRNA. The expression of CXCL10, IL1A and IFNB1 which are SASP-associated genes was analyzed. The hSATII RNA was knocked down by introducing siRNA into SVts8 cells with Lipofectamine RNAiMAX Transfection Reagent (Thermo Fisher Scientific). The following siRNAs were used to perform transfection at a concentration of 20 nM. After 2 days, the expression of each gene was analyzed by RT-qPCR.
hSATII siRNA
   #1: 5'-UUUCCAUUCC AUUCCAUUC-3' (SEQ ID NO: 1)
   #2: 5'-AAUCAUCGAA UGGUCUCGA-3' (SEQ ID NO: 2)
Control
   5'-AUGAACGUGA AUUGCUCAA-3' (SEQ ID NO: 3)

The expression levels of hSATII, CXCL10, IL1A and IFNB1 were analyzed by RT-qPCR. The total RNA was extracted with TRIzol Reagent (Thermo Fisher Scientific), and contaminating DNA was removed by a TURBO DNA-free kit (Applied Biosystems), followed by reverse transcription with PrimeScript RT Master Mix (TaKaRa). The RT-qPCR was performed by a StepOne Plus PCR system (Thermo Fisher Scientific) using SYBR Premix Ex TaqII (TaKaRa). The following sequences were used as primers.
hSATII
   Forward: 5'-AATCATCGAA TGGTCTCGAT-3' (SEQ ID NO: 4)
   Reverse: 5'-ATAATTCCAT TCGATTCCAC-3' (SEQ ID NO: 5)
CXCL10
   Forward: 5'-CCAGAATCGAAGGCCATCAA-3' (SEQ ID NO: 6)
   Reverse: 5'-CATTTCCTTGCTAACTGCTTTCAG-3' (SEQ ID NO: 7)
IL1A
   Forward: 5'-AACCAGTGCTGCTGAAGGA-3' (SEQ ID NO: 8)
   Reverse: 5'-TTCTTAGTGCCGTGAGTTTCC-3' (SEQ ID NO: 9)
IFNB1
   Forward: 5'-AAACTCATGAGCAGTCTGCA-3' (SEQ ID NO: 10)
   Reverse: 5'-AGGAGATCTTCAGTTTCGGAGG-3' (SEQ ID NO: 11)

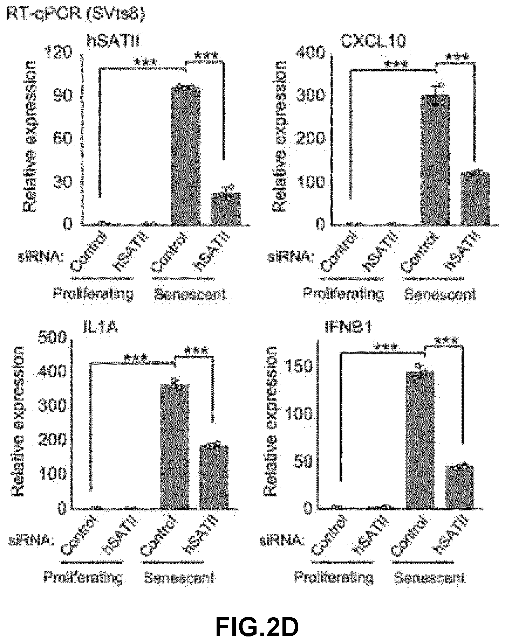

The knockdown of hSATII RNA decreased the expression of CXCL10, IL1A and INFB1 in senescent cells. Figure 2D shows a fold change (FC) as a relative expression level when the expression in proliferative cells treated with control siRNA is 1. Similar results were obtained for senescent cells from passage culture of IMR-90 cells (data not shown). These data suggest that hSATII RNA changes its chromatin accessibility with cellular senescence, whereby the expression of a SASP-like inflammation-associated gene is controlled.

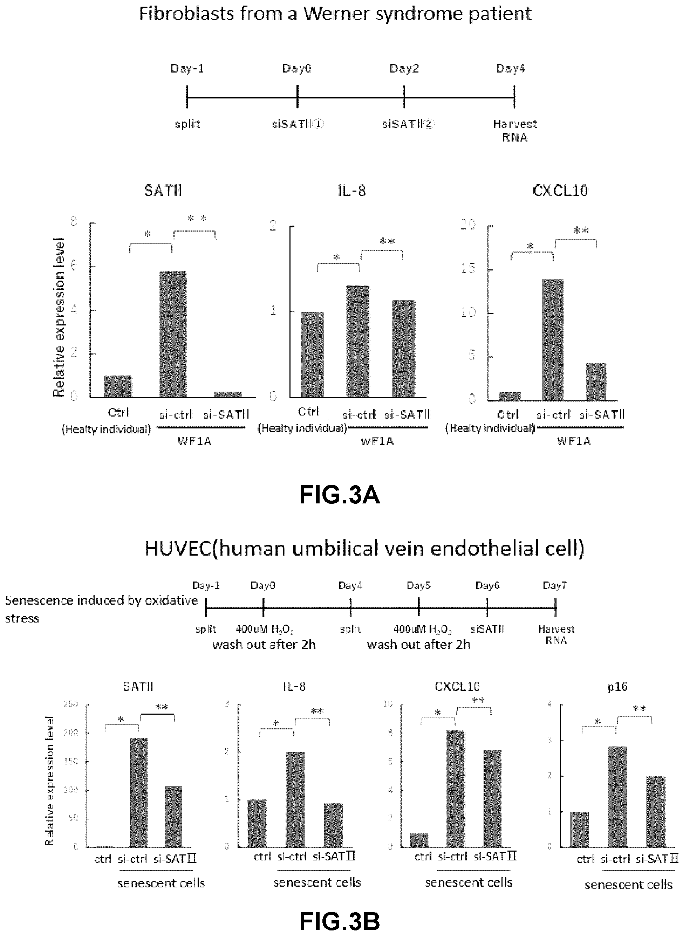

Whether hSATII expression controlled inflammatory gene expression was also analyzed for diseases and disease models caused by cellular senescence. The Werner syndrome is a hereditary disease, as well as one of premature senility syndromes in which various symptoms of aging such as graying hair and cataract appear after adolescence, and various age-related diseases develop, resulting in a short lifetime. The passage day was defined as Day-1. On the 0th day that is the day after passage and the 2nd day, hSATII siRNA was introduced into skin-derived fibroblasts from a Werner syndrome patient (WF1A) and skin-derived fibroblasts from a healthy individual (Ctrl) to suppress SATII expression, and on the 4th day, RNA was extracted from the cells, and the expression of IL-8 and CXCL10 were analyzed by RT-qPCR (Figure 3A). The suppression of hSATII expression resulted in significant suppression of IL-8 and CXCL10 expression.

HUVEC (human umbilical vein endothelial cell) is known as a vascular endothelial cell model in which cellular senescence can be induced by oxidative stress. The passage day was defined as Day-1. On the next day (0th day), 400 µM hydrogen peroxide was added to HUVEC, which was cultured for 2 hours while being placed under oxidative stress. Thereafter, the hydrogen peroxide was removed, and passage was performed again on the 4th day. On the day after the passage (5th day), the cells were cultured with 400 µM hydrogen peroxide for 2 hours, and the hydrogen peroxide was removed. On the next day (6th day), hSATII siRNA was introduced to suppress SATII expression. After 1 day (7th day), RNA was extracted from the cells, and the expression of IL-8, CXCL10 and p16 was analyzed by RT-qPCR (Figure 3B). When cellular senescence was induced by applying oxidative stress, the expression of SATII RNA increased. When the expression was suppressed by siRNA, the expression of SASP-like inflammation-associated genes such as IL-8 and CXCL10, and p16 that is a cellular senescence marker were significantly suppressed. In each case of Werner syndrome in which cellular senescence is considered to occur, and cells in which oxidative stress was induced, suppression of SATII resulted in suppression of the expression of the SASP-like inflammation-associated gene, and therefore, SATII was shown to control the expression of inflammation-associated genes in senescent cells.

### [Mechanism of controlling inflammation-associated gene by hSATII RNA]

An attempt was made to identify hSATII RNA-binding proteins for analyzing the mechanism of promoting the expression of a SASP-like inflammation-associated gene by hSATII RNA. An association between hSATα RNA located in the centromeric region and a specific protein has been reported, but there has not been a report of an association of a specific protein for hSATII RNA located in the pericentromeric region. Thus, RNA pull-down and mass analysis were performed and identified 280 hSATII RNA-binding proteins.

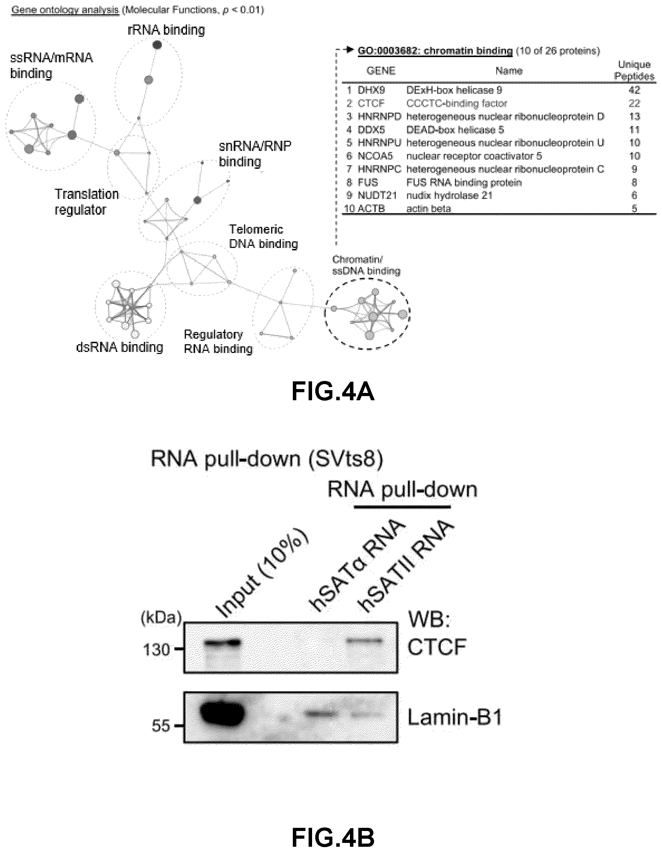

Among these proteins, 26 chromatin-binding proteins (GO: 0003682) were identified by gene ontology analysis, but analysis was performed with a focus on CTCF because it exhibited a high strength score in mass analysis, and was reported to contribute to maintenance of a chromatin steric structure in many cases (Figure 4A). Whether hSATII RNA and CTCF were bound was confirmed by an RNA pull-down assay. The SVts8 cell lysate was analyzed by Western blotting after the RNA pull-down assay. It was confirmed that hSATII RNA was bound to CTCF, but hSATα RNA was not (Figure 4B).

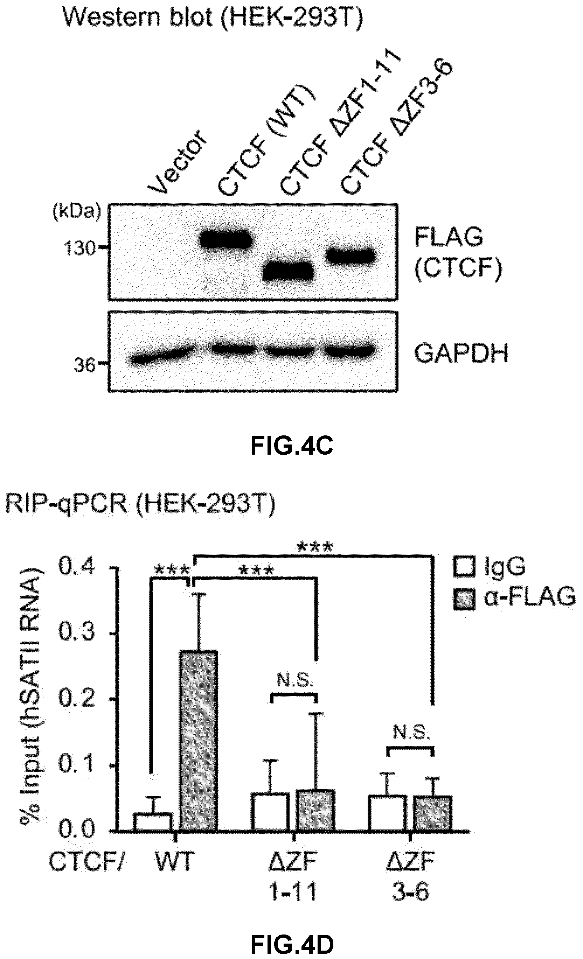

It is known that binding of CTCF to genomic DNA is important for maintaining an appropriate steric structure of a genome. Since CTCF is an RNA-binding protein, RNA immunoprecipitation (RIP) was performed (Figure 4D). Wild-type CTCF (WT), and ZF (Zinc Finger)-deleted mutants of CTCF ΔZF1-11 and ΔZF3-6 were expressed in HEK293T cells (Figure 4C). The hSATII RNA was found to bind to wild-type CTCF, but was not found to bind to the CTCF mutants ΔZF1-11 and ΔZF3-6 (Figure 4D). CTCF has 11 ZF domains, and it is said that binding between ZF1 or ZF10 and RNA is important for forming a chromatin loop to control gene expression. However, although data are not shown, it was found that hSATII RNA did not bind either to ZF1 or to ZF10. It has been revealed that ZF3-ZF6-deleted mutants of CTCF (CTCF ΔZF3-6) known as a DNA-binding domain cannot bind to hSATII RNA, therefore ZF3-ZF6 is important for binding to hSATII RNA.

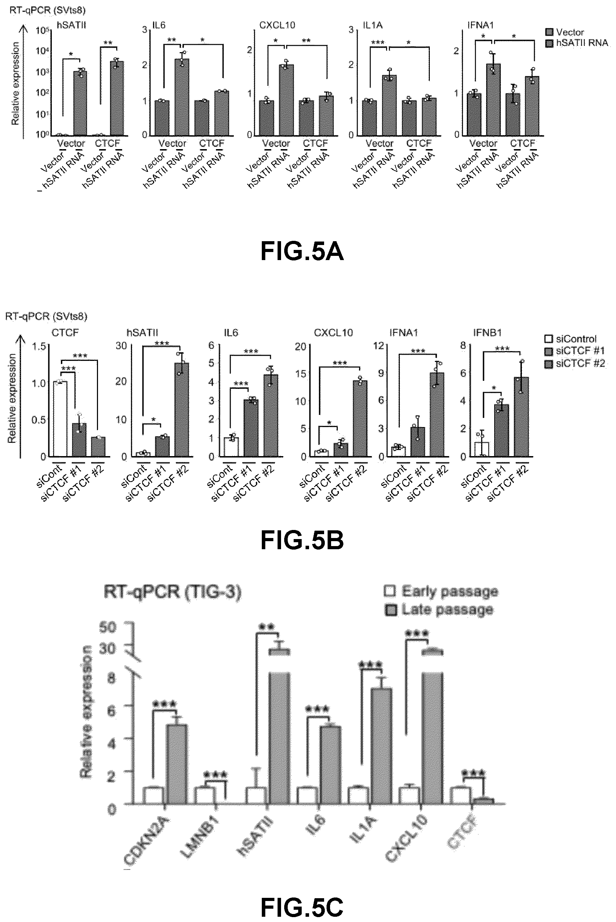

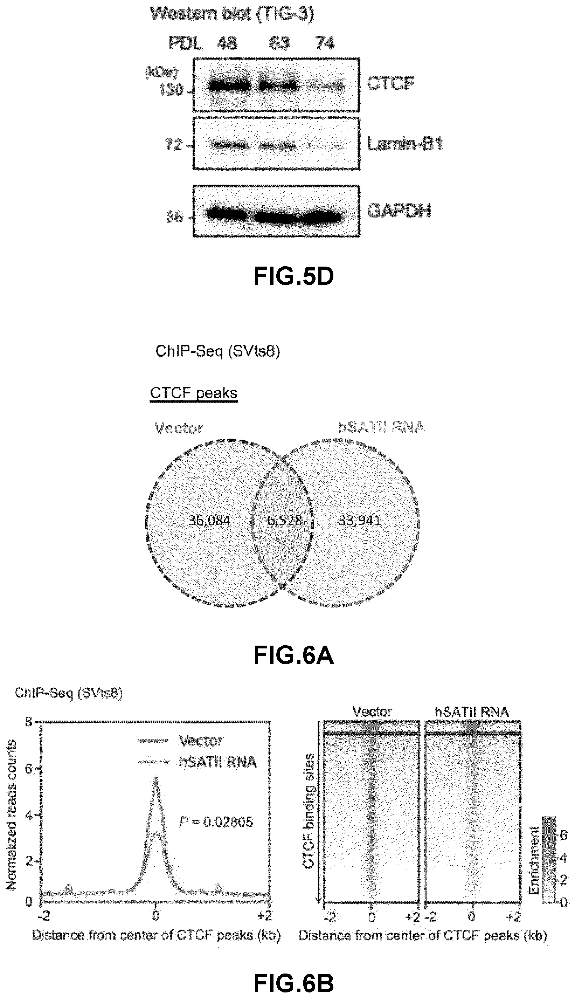

For SVts8 cells in which CTCF was forcibly expressed, and hSATII RNA was overexpressed, the expression of inflammation-associated genes was analyzed by RT-qPCR (Figure 5A). In situations where CTCF was overexpressed, even hSATII RNA was expressed, its effect was eliminated, and the expression of the inflammation-associated gene was not enhanced. Further, in proliferative cells, CTCF was knocked down with CTCF siRNA (Thermo Fisher Scientific, #HSS116455, #HSS116456, Negative Control (Duplex High GC Duplex) siRNA, #46-2000), and the expression of the inflammation-associated genes was analyzed (Figure 5B). The knockdown of CTCF enhanced the expression of the inflammation-associated gene. Unexpectedly, it was revealed that the knockdown of CTCF also increased the expression of hSATII RNA. These results suggested that the expression of inflammation-associated genes which is induced by hSATII RNA was likely to depend on the dysfunction of CTCF, and the CTCF controlled the expression of hSATII RNA in the process of cellular senescence. Then, senescence was induced in TIG-3 cells (obtained from JCRB Cell Bank) through passage culture, and the CTCF expression level was analyzed by RT-qPCR and Western blotting (Figures 5C and 5D). As the passage culture progressed, the expression level of CTCF decreased (Figures 5C and 5D), and the expression levels of hSATII RNA and the inflammation-associated genes increased (Figure 5C). This suggested that CTCF controlled the expression of hSATII RNA in the process of cellular senescence.

Further, although data are not shown, the expression of major satellite (MajSAT) RNA located in the pericentromeric region of mice was analyzed in a manner similar to that for human hSATII RNA. In mouse embryonic fibroblasts (MEF), DNA damage was generated with doxorubicin to induce cellular senescence. As a result, there was an increase in expression of MajSAT RNA as well as SASP-associated inflammation genes. That is, this indicated that the induction of MajSAT RNA was negatively correlated with the expression of CTCF even in the case of mice. Further, CTCF bound not to minor satellite (MinSAT) RNA present in the centromere, but to MajSAT RNA present in the pericentromere of the mice, and increased the expression of SASP-associated inflammation genes. These findings indicated that CTCF was important for controlling the expression of satellite RNA in the pericentromere and SASP-associated inflammation genes in cellular senescence.

### [Functional mechanism of hSATII RNA]

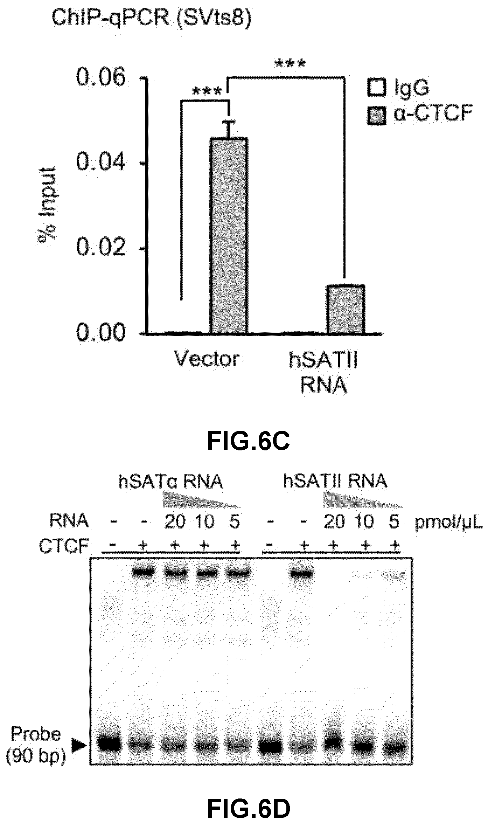

Since the ZF3-ZF6 DNA-binding domain of CTCF was related to binding to hSATII RNA, it was assumed that hSATII RNA directly bound to the ZF domain of CTCF to change the DNA binding ability, and the following studies were conducted. The hSATII RNA was expressed, and a ChIP-Seq assay was performed (Figures 6A and 6B). As expected, the overexpression of hSATII RNA led to a change in distribution of CTCF binding peaks. The ability of CTCF to bind to ICR (imprinting control region) which is a typical binding site of CTCF located between IGF2 and H19 was analyzed by ChIP-qPCR and EMSA (Electrophoretic Mobility Shift Assay) (Figures 6C and 6D). The expression of hSATII RNA reduced the DNA binding ability of CTCF, and it was revealed that the binding was inhibited in a manner dependent on the concentration of RNA of hSATII.

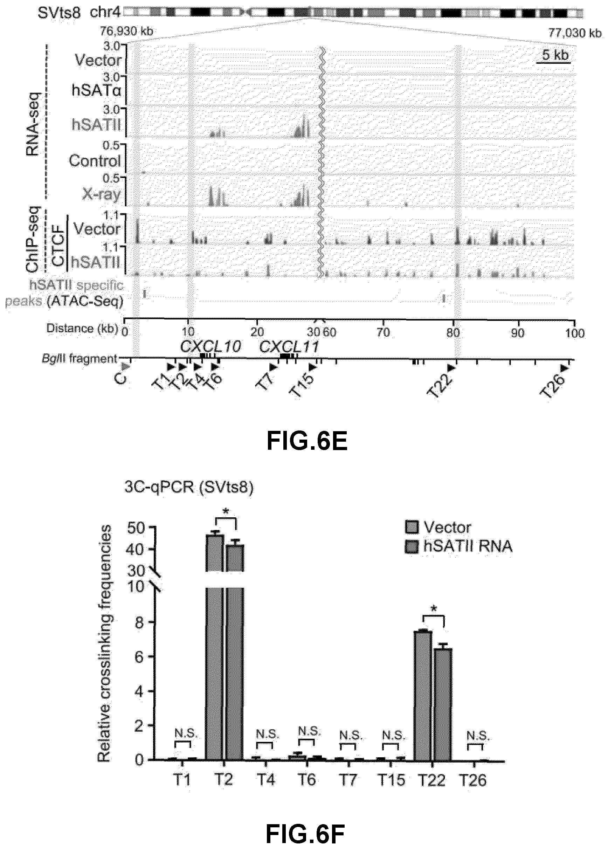

The hSATII RNA changes the interaction with chromatin via binding between CTCF and DNA. This suggests that hSATII RNA may be important for maintaining an appropriate steric structure of a genome. Thus, hSATII RNA was expressed, and a chromosome conformation capture (3C) assay was performed in the vicinity of the gene loci of CXCL10/CXCL11 which are SASP genes. The results revealed that the overexpression of hSATII RNA markedly depressed the interaction in T2 and T22 regions. Further, ATAC-seq analysis showed that chromatin accessibility in the gene loci increased, and there was an increase in expression of SASP-associated inflammation genes. A similar phenomenon was also found in senescent cells induced by an X-ray (Figures 6E and 6F). These results suggested that an increase in expression of hSATII RNA in senescent cells caused a change in conformation of the chromatin structure in SASP gene loci. A molecular mechanism behind the association between control of CTCF and SASP gene expression in cellular senescence had not been clarified, but it became evident that hSATII RNA in pericentromere inhibits the function of CTCF to influence the chromatin interaction, which leads to a change in expression of the SASP-associated inflammation-associated gene.

### [Effect of hSATII RNA on tumor]

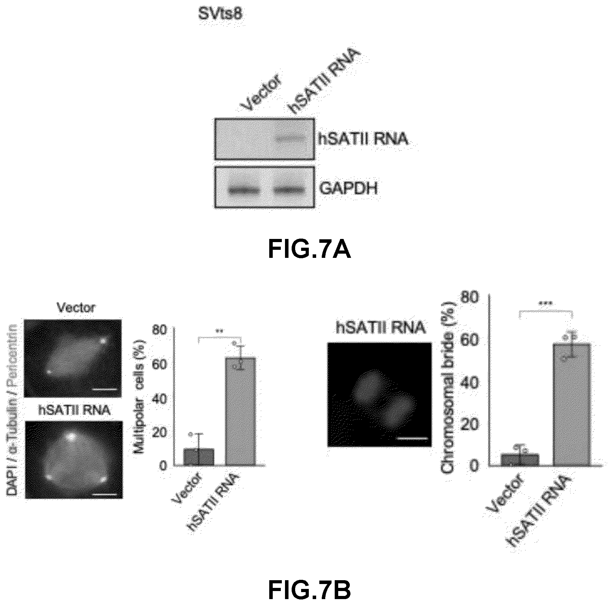

It has been reported that the satellite RNA of humans and mice may induce chromosomal instability, leading to tumor formation. SATII RNA was forcibly expressed in SVts8 cells (Figure 7A), multi-polarization and formation of chromosome bridges, which are typical characteristics of chromosomal instability, were analyzed by immunostaining (Figure 7B). Microtubules were stained with an anti-α-tubulin antibody (Sigma-Aldrich), centrosomes were stained with pericentrin (Abcam), and DNA was stained with DAPI (Thermo Fisher Scientific). The proportion of multipolar cells significantly increased in the cells in which hSATII RNA had been forcibly expressed (Figure 7B, left). The proportion of cells in which chromosome bridges were formed also significantly increased in the cells in which hSATII RNA had been forcibly expressed (Figure 7B, right).

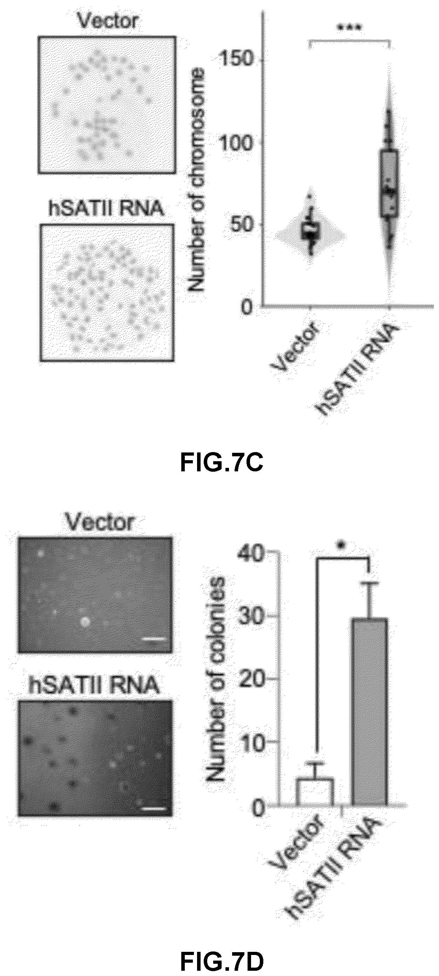

Further, the cells in which hSATII RNA had been forcibly expressed showed phenotypes that are found in tumor cells, such as an abnormal number of chromosomes and scaffold-independent proliferation. Karyotype analysis was performed, and the results showed that the number of cells having an abnormal number of chromosomes significantly increased in the cells in which hSATII RNA had been forcibly expressed (Figure 7C). Whether hSATII RNA expression caused scaffold-independent proliferation was analyzed by a soft agar colony formation assay. The hSATII RNA expression led to a significant increase in the number of colonies that proliferate in a scaffold-independent manner (Figure 7D).

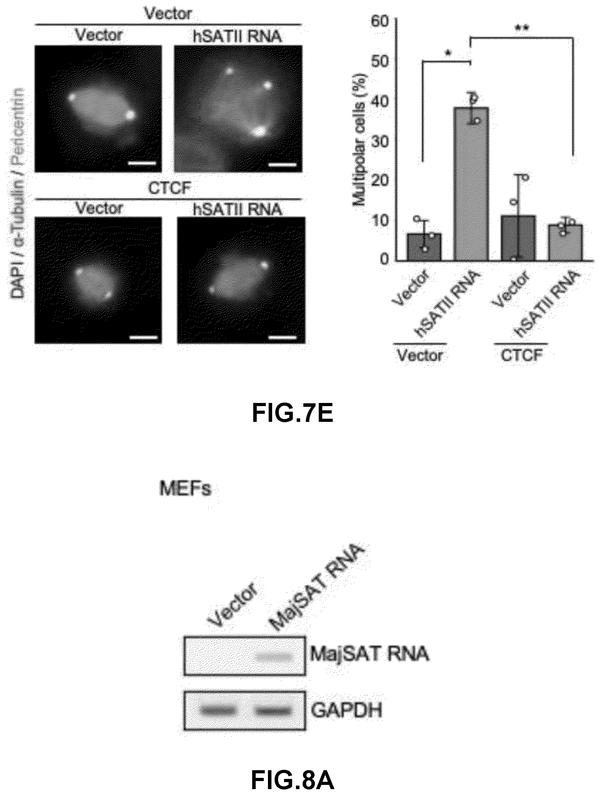

Next, hSATII RNA and CTCF were overexpressed at the same time to examine whether chromosomal instability induced by hSATII RNA was influenced. The hSATII RNA and CTCF were forcibly expressed in SVts8 cells, and the proportion of multipolar cells was analyzed in a manner similar to that in Figure 7B. The results revealed that the expression of CTCF eliminated chromosomal instability induced by hSATII RNA (Figure 7E). This suggested that CTCF was involved in chromosomal instability induced by hSATII RNA, thus being a risk factor for tumor formation.

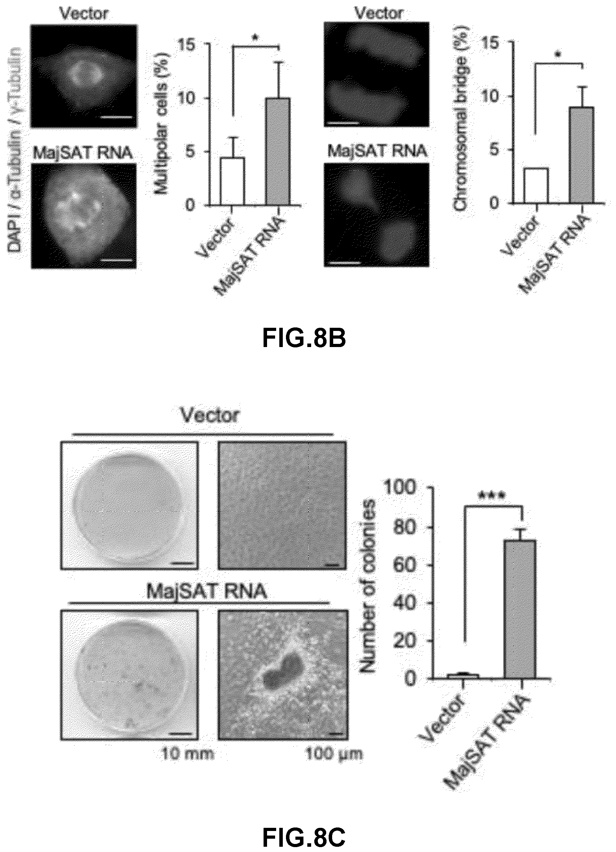

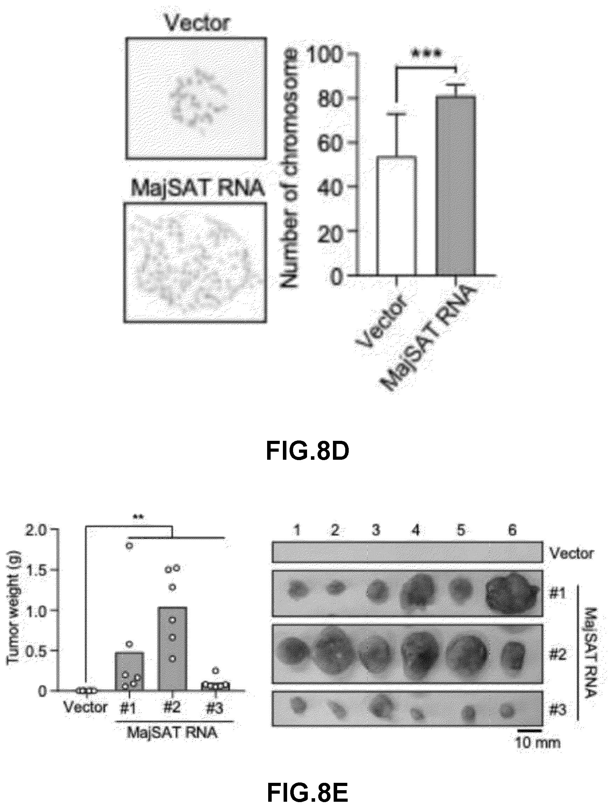

The results of ectopically expressing MajSAT RNA using mouse embryonic fibroblasts (MEFs) are shown. In the same manner as in Figure 7, MajSAT RNA was forcibly expressed in MEFs (Figure 8A), and multi-polarization and formation of chromosome bridges (Figure 8B) were observed with a microscope. They both significantly increased due to the forcible expression of MajSAT RNA. Analysis was also performed on scaffold-independent proliferation (Figure 8C) and the karyotype (Figure 8D), and the results showed that the expression of MajSAT RNA led to a significant increase in the number of transformed cells that proliferate in a scaffold-independent manner, and the abnormality of the number of chromosomes. Further, these cells, which were implanted under the skin of nude mice, exhibited a tumorigenic potential (Figure 8E). Therefore, it is concluded that satellite RNA in the pericentromere may promote cancer development.

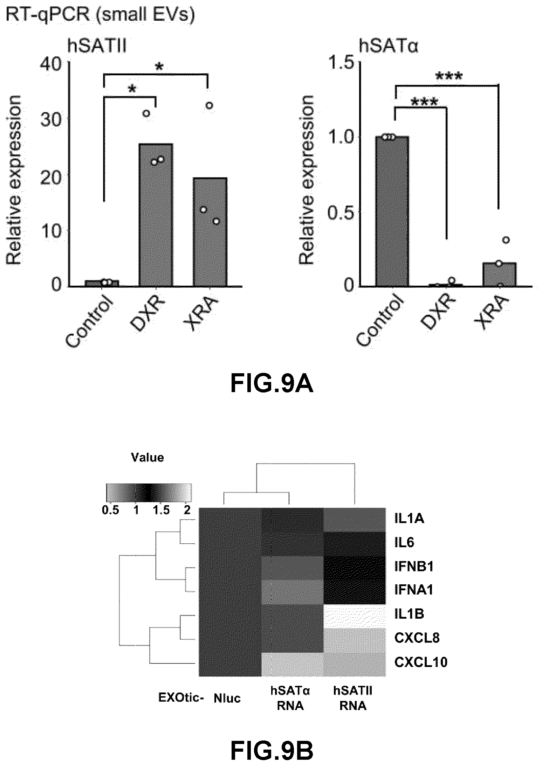

Next, the functions of hSATII RNA in microenvironment of tumors were given attention, and analyzed. Extracellular vesicles secreted from cancer cells and senescent stromal cells are known to be involved in the development and progress of tumors in tumor microenvironment. Doxorubicin (DXR) or X-ray radiation (XRA) at 40-gray was applied to RPE-1/hTERT cells (obtained from ATCC) to induce cellular senescence in the cells. Extracellular vesicles were collected from these cells, and subjected to RT-qPCR, and the results showed that the extracellular vesicles derived from senescent cells contained a significantly larger amount of hSATII RNA than extracellular vesicles derived from proliferative cells. However, there was no increase in the amount of hSATα RNA (Figure 9A).

It has been reported, based on analysis of RNA-seq data, that hSATII RNA is detected from extracellular vesicles (exosomes) secreted from a human colorectal cancer cell line. These lead to an inference that hSATII RNA derived from senescent stromal cells moves to peripheral cells through extracellular vesicles, and functions as a SASP-like inflammation factor. Although data are not shown, the present inventors have confirmed that extracellular vesicles derived from senescent cells induce scaffold-independent proliferation and chromosomal instability in normal cells. For evaluating the involvement of hSATII RNA contained in extracellular vesicles, designer exosomes containing hSATII RNA were established by a synthetic biological method using an EXOtic device (EXOsomal transfer into cells) (Non Patent Literature 12). The prepared designer exosomes were introduced into SVts8 cells, and expression of inflammation-associated genes was analyzed by RT-qPCR. The designer exosomes containing hSATII RNA were found to promote the expression of SASP-like inflammation-associated genes (Figure 9B). That is, the prepared designer exosomes were found to have oncogenic activity similar to that of extracellular vesicles derived from senescent cells. This indicates that hSATII RNA contained in exosomes secreted from senescent cells promotes SASP-like inflammation-associated gene expression and chromosomal instability in stromal cells in tumor microenvironment.

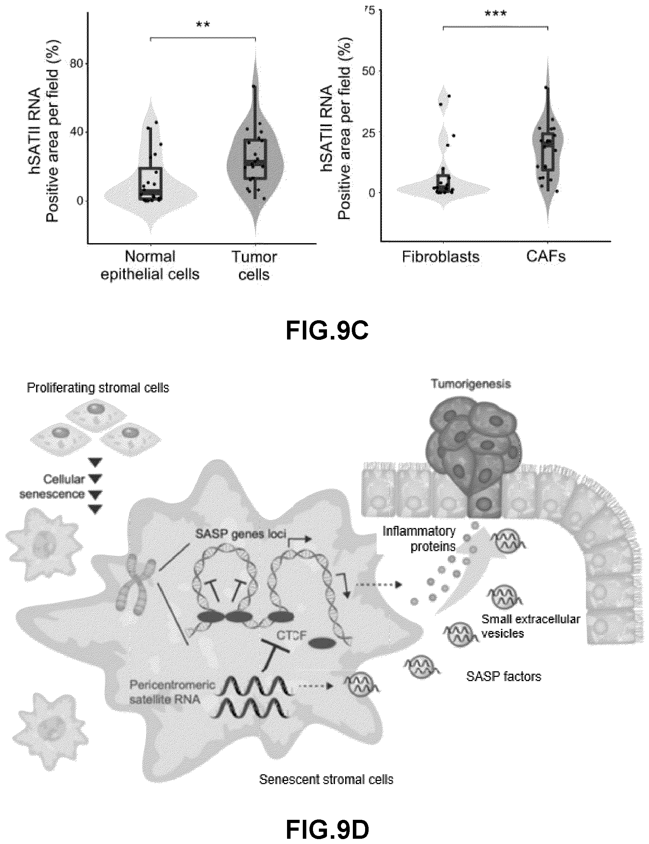

The expression of hSATII RNA in tumor microenvironment was analyzed by an RNA in situ hybridization (RNA-ISH) method using surgical resection samples from primary colorectal cancer patients. The results showed that the number of colorectal cancer cells in which hSATII RNA was expressed was significantly larger than the number of normal epithelial cells (Figure 9C, left). For the cancer-associated fibroblasts, the proportion of SATII RNA-positive cells was significantly higher than that of fibroblasts of normal stromal tissues (Figure 9C, right). This suggests that senescent stromal cells which express hSATII RNA promote tumor formation in tumor microenvironment via secretion of extracellular vesicles containing SASP-like inflammation factors and hSATII RNA (Figure 9D).

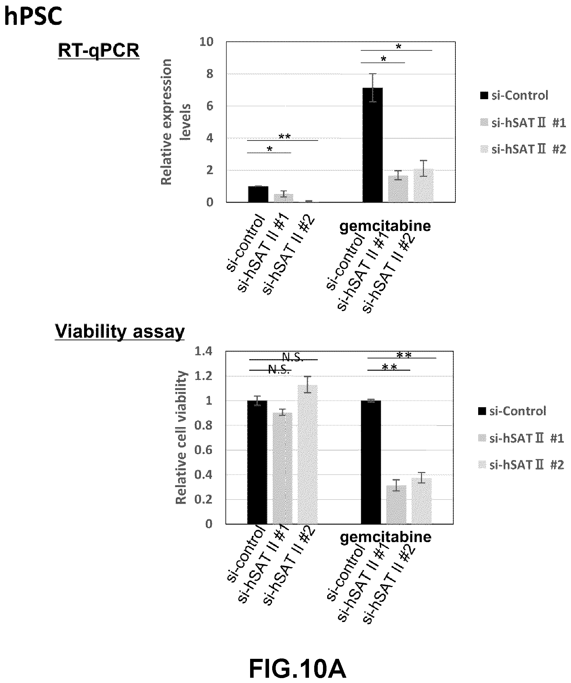

As described above, it is considered that senescent stromal cells that express RNA may form tumor microenvironment and promote tumor formation. Thus, analysis was performed on whether suppression of the expression of hSATII had an effect on suppression of proliferation of stromal cells. The siRNA of hSATII was introduced into normal pancreatic stromal cells hPSC (obtained from ScienCell), followed by analysis of expression by RT-qPCR. Gemcitabine was added to a culture medium to induce cellular senescence, resulting in increased expression of hSATII RNA (Figure 10A, upper side). The cell viability assay, CellTiter-Glo (Promega), performed at the same time showed that normal pancreatic stromal cells were not influenced by hSATII knockdown, but in pancreatic stromal cells in which cellular senescence was induced with gemcitabine, suppression of the expression of hSATII RNA by siRNA induced cell death (Figure 10A, lower side). These results indicate that when an anticancer agent is administered, and the expression of hSATII RNA is suppressed, cell death is induced not only in cancer cells, but also in stromal cells forming tumor microenvironment.

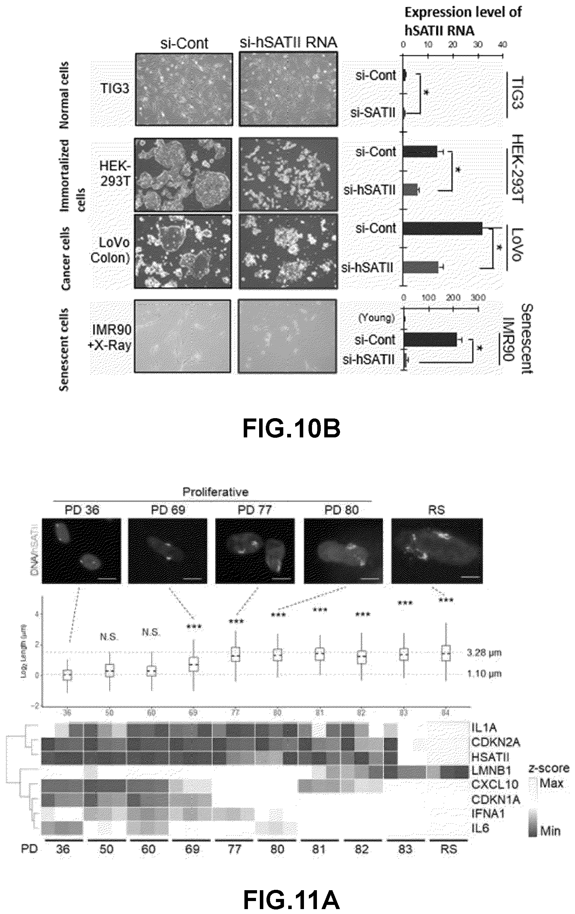

As will be described below, suppression of the expression of hSATII RNA induces cell death or suppression of proliferation not only in cancer cells, but also in senescent cells and immortalized cells. In senescent cells obtained by inducing cellular senescence in normal cells TIG3, immortalized cells HEK-293T, colorectal cancer cells LoVo, and IMR90 being normal cells by X-ray irradiation, hSATII expression was suppressed by siRNA. The microphotographs of the cells 3 days after the introduction of siRNA are shown (Figure 10B). While proliferation of TIG-3 cells being normal cells did not change, proliferation of HEK-293T cells, and IMR-90 cells in which senescence had been induced was obviously suppressed, and it was found that cell death was induced in LoVo cells. These results show that by suppressing the expression of hSATII RNA, cell death is induced in senescent cells to eliminate the senescent cells, or proliferation of immortalized cells and cancer cells is suppressed. This indicates that suppression of hSATII RNA expression may enable treatment diseases caused by cellular senescence.

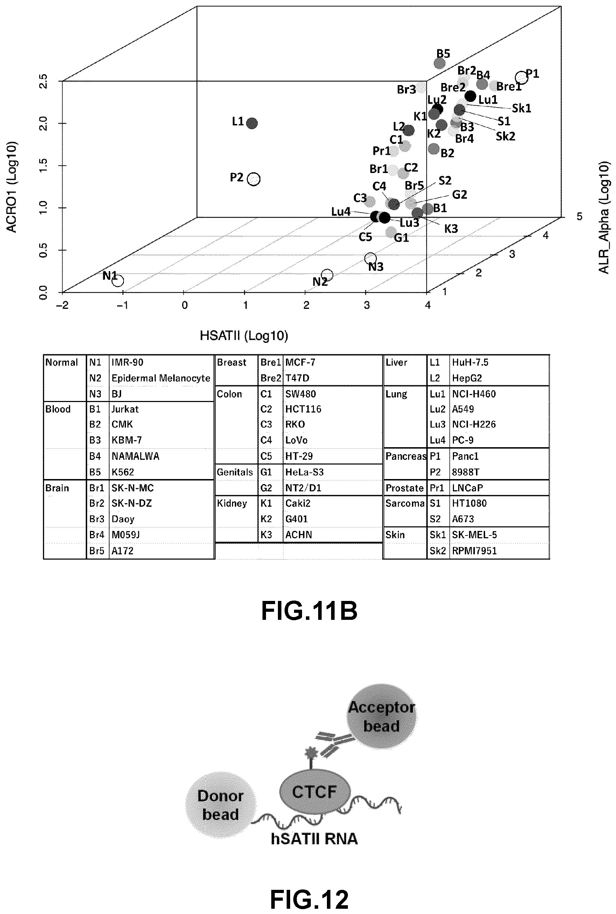

The present inventors found that the hSATII DNA region swelled before the expression of hSATII RNA by DNA-FISH analysis of the hSATII DNA region in the process of inducing cellular senescence into TIG-3 cells through passage culture (Figure 11A). Although DNA-FISH was performed using a probe of SEQ ID NO: 12, any sequence may be used as long as the probe is capable of detecting the hSATII DNA region. Further, the chromatin accessibility of the hSATII DNA region was analyzed from a DNaseI sequence data of a plurality of cancer cell lines, and the results showed that the chromatin accessibility in cancer cells evidently increased as compared to that in normal fibroblasts (Figure 11B).

As is apparent from the above results, cellular senescence and associated diseases can be suppressed or prevented by decreasing hSATII RNA which is non-coding RNA in the pericentromere. Specifically, by administering a drug that suppresses hSATII RNA expression, senescent cells can be eliminated, and secretion of SASP factors can be suppressed. As has been described above, a substance that targets hSATII RNA expression and suppresses the expression can become a new therapeutic drug. Specifically, as shown above, nucleic acid medicines such as siRNA and LNA enables a decrease in hSATII RNA. For example, siRNAs represented by SEQ ID NOS: 1 and 2 are useful because they enable hSATII RNA to disappear with good efficiency. In addition, new LNA and siRNA may be designed, and used. Further, compounds that target hSATII RNA expression can be screened, and used.

Whether the disease is caused by cellular senescence can be confirmed by examining hSATII RNA expression. For example, the use of a set of primers represented by SEQ ID NOS: 4 and 5 enables confirmation of hSATII RNA expression. It is also possible to use a tissue sample for detection by RNA-ISH. Since hSATII RNA is contained in extracellular vesicles such as exosomes, it is also possible to detect the amount of hSATII RNA in extracellular vesicles using body fluids such as blood, urine and ascitic fluid. SASP factors, the expression of which is promoted by hSATII RNA expression, may be measured. For SASP factors, RNA expression may be measured, or the amount of protein or the amount of secretion may be measured by ELISA. Samples used may be tissues of affected parts, for example, tissues taken through biopsy in the case of cancer. Extracellular vesicles derived from body fluid may be used. A decrease in CTCF expression may be examined. If expression of hSATII RNA, expression of a SASP factor, or decreased expression of CTCF is recognized, the pharmaceutical composition that suppresses hSATII RNA expression described above can be used for treatment. Since the hSATII DNA region swells and has increased chromatin accessibility before the expression of hSATII RNA, it may be possible to predict/diagnose a disease by epigenomic analysis such as DNA-FISH analysis or ATAC sequence analysis, or analysis using genomic DNA fragments or extracellular vesicles in the body fluid.

It is also possible to screen compounds using suppression of expression of hSATII RNA, suppression of expression of SASP factors, increased expression of CTCF, or an epigenomic change such as shrinkage or decreased chromatin accessibility of the hSATII DNA region as an index. For example, by constructing a FRET-based system, low-molecular compounds can be screened with high sensitivity. Specifically, system can be constructed in which a bead bound to hSATII RNA labeled with fluorescent substance as a donor and a bead labeled with fluorescent substance which is bound to CTCF with a specific binding substance such as an antibody as an acceptor (Figure 12). By adding a library compound, followed by excitation at a specific fluorescence wavelength, whether SATII RNA and CTCF are close to each other can be determined. Alternatively, when in culture of senescent cells with a candidate compound, the candidate compound causes suppression of hSATII RNA expression, increased expression of CTCF, or shrinkage or decreased chromatin accessibility of the hSATII DNA region, the compound can function as a therapeutic drug for a disease caused by aging. For the senescent cells, cellular senescence induction methods commonly used in the field, such as passage culture, X-ray irradiation and doxorubicin exposure described in Examples, can be used. The expression of hSATII RNA and CTCF can be analyzed by well-known methods such as RT-qPCR, RNA-ISH. The expression can be monitored by analysis of gene expression and secretion of SASP factors.

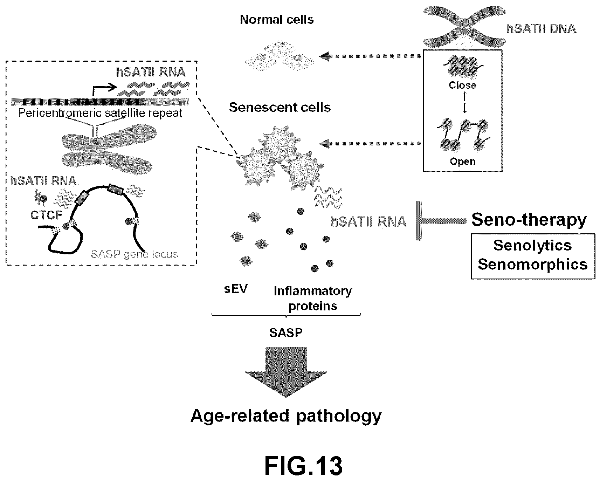

As described above, the mechanism of inducing SASP by hSATII RNA, and its involvement in viability of senescent cells have been clarified. In senescent cells, the pericentromeric region of the chromosome is opened, and from the region, hSATII RNA which is non-coding RNA is actively transcribed. It has been discovered that by binding to CTCF having a function important for maintaining chromosomal structures, hSATII RNA inhibits the function to change chromosome interaction, so that transcription of inflammatory genes is induced. Senescent cells in which inflammatory genes are actively transcribed are involved in development of age-related diseases and exacerbation of pathological conditions. Therefore, it may be possible to prevent or treat age-related diseases by Seno-therapy having both the Senomorphic function of recognizing hSATII RNA as a new target and suppressing the expression of inflammatory SASP-associated genes, and the Senolytic function of selectively inducing cell death in senescent cells. Since hSATII RNA expression, CTCF expression and the epigenomic change of genomic DNA occur in the early stage of cellular senescence, detection thereof enables early detection of senescent cells that cause age-related diseases (Figure 13).

## Claims

1. A pharmaceutical composition which is a therapeutic drug and/or a prophylactic drug for a disease caused by senescent cells, the pharmaceutical composition suppressing expression or inhibiting activity of hSATII RNA, and/or increasing expression of CTCF.

2. The pharmaceutical composition according to claim 1, wherein the pharmaceutical composition is a nucleic acid medicine which suppresses the expression or inhibits the activity of hSATII RNA and/or increases the expression of CTCF.

3. A method for acquiring data on whether cellular senescence occurs in a subject, comprising detecting any one of hSATII RNA, CTCF expression, and an epigenomic change of genomic DNA in a sample.

4. The method for acquiring data according to claim 3, wherein a disease caused by senescent cells is used in a test.

5. A method for screening a therapeutic drug and/or a prophylactic drug for a disease caused by cellular senescence, comprising
adding a candidate compound to a medium for senescent cells,
the candidate compound being selected at least one selected from the group of decreased expression of hSATII RNA, inhibition of functions of hSATII RNA, increased expression of CTCF, decreased expression of a SASP factor, shrinkage of the hSATII DNA region and decreased chromatin accessibility as an index.
